# EUROPEAN PATENT SPECIFICATION

(11) **EP 3 646 863 B1**
(45) Date of publication and mention of the grant of the patent: **03.01.2024**
(21) Application number: 18823051.0
(22) Date of filing: 29.06.2018
(51) Int. Cl.: A61K 31/423, A61K 9/20, A61K 47/02, A61K 47/10, A61K 47/20, A61K 47/26, A61K 47/32, A61K 47/36, A61K 47/38, A61P 1/16, A61P 3/06, A61K 9/14, A61K 9/16

(54) **PHARMACEUTICAL COMPOSITION**
PHARMAZEUTISCHE ZUSAMMENSETZUNG
COMPOSITION PHARMACEUTIQUE

(30) Priority: 30.06.2017 JP 2017128722
(43) Date of publication of application: 06.05.2020
(73) Proprietor: Kowa Company, Ltd., Nagoya-shi, Aichi 460-8625 (JP)
(72) Inventor: SUGIMOTO, Shin, Fuji-shi Shizuoka 417-8650 (JP); MINAMIZONO, Akito, Fuji-shi Shizuoka 417-8650 (JP)
(74) Representative: Blodig, Wolfgang
(86) International application number: PCT/JP2018/024885
(87) International publication number: WO 2019/004450

(56) References cited:
- EP-A1- 1 661 890
- EP-A1- 2 902 025
- EP-A1- 3 020 401
- EP-B1- 1 661 890
- EP-B1- 2 902 025
- EP-B1- 3 020 401
- WO-A1-2005/023777
- WO-A1-2014/050134
- WO-A1-2015/005365
- WO-A1-2017/082377
- AM ENDE MARY T ET AL: "Improving the content uniformity of a low-dose tablet formulation through roller compaction optimization", PHARMACEUTICAL DEVELOPMENT AND TECHNOLOGY, NEW YORK, NY, US, vol. 12, no. 4, 1 January 2007 (2007-01-01), pages 391-404, XP008166169, ISSN: 1083-7450, DOI: 10.1080/10837450701369253
- Vipin Kukkar ET AL: "Mixing and formulation of low dose drugs: underlying problems and solutions", THAI JOURNAL OF PHARMACEUTICAL SCIENCES, 31 December 2008 (2008-12-31), pages 43-58, XP055012249, Retrieved from the Internet: URL:http://www.thaiscience.info/journals/A rticle/Mixing [retrieved on 2011-11-16]
- Anonymous: "Report on the deliberation results of parmodia tablets 0.1 mg", Pharmaceutical Evaluation Division of Pharmaceutical Safety and Environmental Health Bureau, 17 May 2017 (2017-05-17), XP009518270,

## Description

### Field of the Invention

The present invention relates to a pharmaceutical composition etc.

### Background of the Invention

It is known that pemafibrate (Chemical Name: (2R)-2-[3-([1,3-Benzoxazol-2-yl[3-(4-methoxyphenoxy)propyl] amino]methyl)phenoxy]butanoic acid)(International Nonproprietary Name: pemafibrate) represented by the following structural formula: 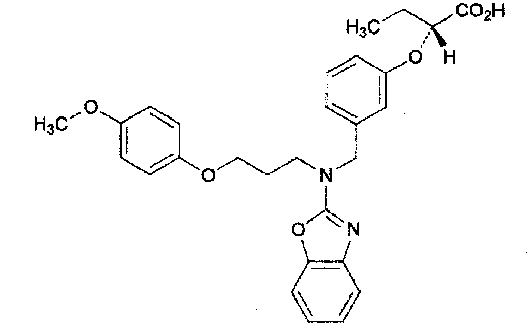 , a salt thereof or a solvate thereof has excellent PPAR-α agonist activity, exhibits plasma triglyceride concentration reducing action, HDL cholesterol increasing action, etc., and is useful for prevention and treatment of dyslipidemia (hyperlipidemia) (Patent Document 1 and Non-Patent Documents 1 and 2), and useful for prevention and treatment of NAFLD (non-alcoholic fatty liver disease) (Patent Document 2).

Meanwhile, a compound useful as an active component for a pharmaceutical preparation is normally formulated as some pharmaceutical composition and supplied, and from the viewpoint of reliably exhibiting expected drug efficacy and avoiding unanticipated adverse side effects, it is very important that the pharmaceutical composition to be supplied maintains a certain level of quality without variations such as lot-to-lot variation.

EP2902025A1 provides a pharmaceutical combination composition and drug combinations for the prevention and/or treatment of dyslipidemic conditions such as atherosclerosis, hypercholesterolemia, low HDL blood disease in mammals including humans. The pharmaceutical composition comprises (a) a compound such as pemafibrate or a salt thereof or a solvate thereof and (b) a cholesterol absorption inhibitor. As a specific preparation example, pemafibrate (0.05 mg, 1part by mass) formulated into a tablet comprising microcrystalline cellulose (37.25 mg, corresponds to 745 parts by mass), modified food grade cornstarch (37.25 mg, corresponds to 745 parts by mass), and magnesium stearate (0.5 mg, 10 parts by mass)is disclosed. The kinds and amounts of (B-1), (B-2), (B-3), (B-4), (B-5), or (B-6) as defined in claim 1 are not mentioned in the document.

EP1661890A1 discloses pemafibrate or a salt thereof, or a solvate thereof formulated alone or with any other pharmacologically acceptable additives into tablets, capsules, granules, powders wherein the additives are selected from diluents includind lactose, sodium chloride, glucose, starch, microcrystalline cellulose, and silicic acid and/or binders including water, ethanol, propanol, simple syrup, liquefied gelatin, hydroxypropyl cellulose, methyl cellulose, ethyl cellulose, shellac, calcium phosphate, and polyvinyl pyrrolidone or disintegrants including agar powder, sodium hydrogencarbonate, sodium lauryl sulfate, and monoglyceryl stearateand/or lubricants including purified talc, stearate salt, borax, and polyethylene glycol. The kinds and amounts of (B-1), (B-2), (B-3), (B-4), (B-5), or (B-6) as defined ni claim 1are not mentioned in D3.

WO2017/082377 discloses pemafibrate or a salt thereof, or a solvate thereof formulated alone or with any other pharmacologically acceptable carrier into tablets, capsules, granules, powders wherein the carriers are selected from starch, mannitol, and lactose; binders such as carboxymethyl cellulose sodium and hydroxypropyl cellulose; disintegrators such as crystalline cellulose and carboxymethyl cellulose calcium. The kinds and amounts of (B-1), (B-2), (B-3), (B-4), (B-5), or (B-6) as defined in claim 1 are not mentioned in the document.

### Prior Art Documents

### Patent Documents

Patent Document 1: International Publication No. WO 2005/023777
Patent Document 2: International Publication No. WO 2015/005365 (also published as EP3020401A1)

### Non-Patent Documents

Non-Patent Document 1: Yukiyoshi Yamazaki, et al., Synthesis, 2008(7), 1017-1022.
Non-Patent Document 2: Fruchart JC., Cardiovasc Diabetol., 2013; 12: 82.

### Summary of the Invention

### Problems to be Solved by the Invention

However, manufacturability of pharmaceutical compositions, such as homogeneity, significantly depends on the physical and chemical properties of components, but it is often impossible to predict such properties from the chemical structures of the components, and there are not a few cases where a problem becomes evident only when a pharmaceutical composition is actually produced. Thus, establishment of a technique for securing homogeneity of a pharmaceutical composition commonly requires considerable try and error.

Pemafibrate, a salt thereof or a solvate thereof has been only reported to exhibit the above-described pharmacological effects, and has heretofore not been specifically studied in terms of a pharmaceutical composition, and manufacturability such as homogeneity of the pharmaceutical composition has heretofore not been reported at all.

In these circumstances, for developing a pharmaceutical composition containing pemafibrate, a salt thereof or a solvate thereof, the present inventors have first actually produced the pharmaceutical composition. As a result, it was found that pharmaceutical compositions becomes different in content of pemafibrate, leading to development of problems with homogeneity (uniformity) of the content of pemafibrate. If pharmaceutical compositions significantly differ in content of pemafibrate, there may be variations in efficacy and safety among the pharmaceutical compositions.

Thus, an object of the present invention is to provide a pharmaceutical composition containing pemafibrate, a salt thereof or a solvate thereof, and having excellent homogeneity.

### Means for Solving the Problems

In order to solve the problem with the content uniformity of pemafibrate, a salt thereof or a solvate thereof in a pharmaceutical composition, the present inventors have further extensively conducted studies, and found that by further incorporating any of the following components 1 to 6 (hereinafter, components 1 to 6 are sometimes referred to as "component (B-1)", "component (B-2)", "component (B-3)", "component (B-4)", "component (B-5)" and "component (B-6)", respectively, and "one or more selected from the group consisting of components (B-1) to (B-6)" is sometimes referred to as "component (B)"):
(1) one or more cellulose ether species selected from the group consisting of methylcellulose, ethylcellulose, hydroxypropylcellulose, hypromellose, carmellose, carmellose potassium, carmellose calcium, carmellose sodium and croscarmellose sodium, and the total content of the cellulose ether species with respect to 1 part by mass of a free form of pemafibrate is 3 to 200 parts by mass;
(2) one or more starch species selected from the group consisting of starch, hydroxypropyl starch, carboxymethyl starch and a salt thereof, and the total content of the starch species with respect to 1 part by mass of a free form of pemafibrate is 5 to 400 parts by mass;
(3) one or more povidone species selected from the group consisting of povidone and crospovidone, and the total content of the povidone species with respect to 1 part by mass of a free form of pemafibrate is 1 to 200 parts by mass;
(4) one or more silicic acid compounds selected from the group consisting of hydrous magnesium silicate, hydrated silicon dioxide and light anhydrous silicic acid, and the total content of the silicic acid compounds with respect to 1 part by mass of a free form of pemafibrate is 1 to 200 parts by mass;
(5) one or more polyhydric alcohols selected from the group consisting of macrogol, erythritol, xylitol, mannitol, sorbitol, maltitol and lactitol, and the total content of the polyhydric alcohols with respect to 1 part by mass of a free form of pemafibrate is 1 to 2,000 parts by mass; and
(6) one or more alkyl sulfate esters selected from the group consisting of a lauryl sulfate ester salt, tetradecyl sulfate ester salts, hexadecyl sulfate ester salts and octadecyl sulfate ester salts, and the total content of the alkyl sulfate esters with respect to 1 part by mass of a free form of pemafibrate is preferably 1 to 200 parts by mass, in a pharmaceutical composition comprising pemafibrate, a salt thereof or a solvate thereof (hereinafter, sometimes referred to simply as "component (A)"), the content uniformity of pemafibrate in the pharmaceutical composition is improved. The present invention has been accomplished on the basis of this finding.

Accordingly, the present invention provides a pharmaceutical composition comprising the following components (A) and (B):
(A) pemafibrate, a salt thereof or a solvate thereof; and
(B) one or more selected from the group consisting of the following components (B-1) to (B-6):
   (B-1) one or more cellulose ether species selected from the group consisting of methylcellulose, ethylcellulose, hydroxypropylcellulose, hypromellose, carmellose, carmellose potassium, carmellose calcium, carmellose sodium and croscarmellose sodium, and the total content of the cellulose ether species with respect to 1 part by mass of a free form of pemafibrate is 3 to 200 parts by mass;
   (B-2) one or more starch species selected from the group consisting of starch, hydroxypropyl starch, carboxymethyl starch and a salt thereof, and the total content of the starch species with respect to 1 part by mass of a free form of pemafibrate is 5 to 400 parts by mass;
   (B-3) one or more povidone species selected from the group consisting of povidone and crospovidone, and the total content of the povidone species with respect to 1 part by mass of a free form of pemafibrate is 1 to 200 parts by mass;
   (B-4) one or more silicic acid compounds selected from the group consisting of hydrous magnesium silicate, hydrated silicon dioxide and light anhydrous silicic acid, and the total content of the silicic acid compounds with respect to 1 part by mass of a free form of pemafibrate is 1 to 200 parts by mass;
   (B-5) one or more polyhydric alcohols selected from the group consisting of macrogol, erythritol, xylitol, mannitol, sorbitol, maltitol and lactitol, and the total content of the polyhydric alcohols with respect to 1 part by mass of a free form of pemafibrate is 1 to 2,000 parts by mass; and
   (B-6) one or more alkyl sulfate esters selected from the group consisting of a lauryl sulfate ester salt, tetradecyl sulfate ester salts, hexadecyl sulfate ester salts and octadecyl sulfate ester salts, and the total content of the alkyl sulfate esters with respect to 1 part by mass of a free form of pemafibrate is preferably 1 to 200 parts by mass, wherein the pharmaceutical composition is a solid preparation.

The present invention also provides a method for improving the content uniformity of pemafibrate, a salt thereof or a solvate thereof in a pharmaceutical composition, the method including the step of incorporating one or more selected from the group consisting of components (B-1) to (B-6) in a pharmaceutical composition containing pemafibrate, a salt thereof or a solvate thereof.

### Effects of the Invention

According to the present invention, it is possible to provide a pharmaceutical composition having improved content uniformity of pemafibrate in the pharmaceutical composition and having excellent homogeneity.

### Detailed Description of the Invention

### <Pemafibrate, salt thereof or solvate thereof (Component (A))>

Herein, "pemafibrate, a salt thereof or a solvate thereof" includes pemafibrate (Chemical Name: (2R)-2-[3-([1,3-Benzoxazol-2-yl[3-(4-methoxyphenoxy)propyl] amino]methyl)phenoxy]butanoic acid) (International Nonproprietary Name: pemafibrate) itself, a pharmaceutically acceptable salt of pemafibrate and a solvate of pemafibrate or a pharmaceutically acceptable salt thereof with water, alcohol (for example ethanol) or the like. The pharmaceutically acceptable salt is not particularly limited, and examples thereof include acid addition salts and base addition salts. Specific examples of the acid addition salts include acid addition salts with inorganic acids, such as hydrochlorides, hydrobromides, hydroiodides, sulfate salts, nitrate salts and phosphate salts; and acid addition salts with organic acids, such as benzoate salts, methanesulfonate salts, ethanesulfonate salts, benzenesulfonate salts, p-toluenesulfonate salts, maleate salts, fumarate salts, tartrate salts, citrate salts and acetate salts. Specific examples of the base addition salts include metal salts such as sodium salts, potassium salts, lithium salts, calcium salts and magnesium salts; salts with amines such as ammonia, trimethylamine, triethylamine, pyridine, collidine and lutidine; and base addition salts with organic bases such as lysine, arginine, cinchonine and cinchonidine.

The shape, the size and the like of pemafibrate, a salt thereof or a solvate thereof are not particularly limited, and when the average particle diameter of primary particles is measured in accordance with The Japanese Pharmacopoeia, 17th Edition, Laser Diffraction Measurement of Particle Size, d50 and d90 values are preferably as follows.
d50: preferably 100 µm or less, more preferably 50 µm or less, still more preferably 20 µm or less, particularly preferably 1 to 20 µm.
d90: preferably 200 µm or less, more preferably 135 µm or less, still more preferably 80 µm or less, particularly preferably 1 to 80 µm.

Pemafibrate, a salt thereof or a solvate thereof is a known compound, and can be produced through a method as disclosed in Patent Document 1, Non-Patent Document 1 or U.S. Patent No. 7,109,226, for example. In the present invention, a pemafibrate crystal which can be produced through the method described in Non-Patent Document 1 (preferably a crystal having a melting point of 95 to 101°C, particularly preferably 97 to 100°C in measurement performed in accordance with The Japanese Pharmacopoeia, 17th Edition, Melting Point Determination Method 1) is preferably used.

The content of pemafibrate, a salt thereof or a solvate thereof in the pharmaceutical composition is not particularly limited, and can be determined in appropriate consideration of the target disease, the type of preparation, the sex, age and symptoms of a patient in need of the composition, and the like. For example, the content can be set so that the daily dose of pemafibrate, a salt thereof or a solvate thereof may be 0.05 to 0.8 mg, more preferably 0.075 to 0.6 mg, particularly preferably 0.1 to 0.4 mg, in terms of a free form of pemafibrate.

The content of pemafibrate, a salt thereof or a solvate thereof in the pharmaceutical composition is preferably 0.01 to 5 mass%, more preferably 0.025 to 1 mass%, particularly preferably 0.05 to 0.5 mass%, in terms of a free form of pemafibrate, with respect to the total mass of the pharmaceutical composition. According to the present invention, even if pemafibrate, a salt thereof or a solvate thereof has such a small content, a good content uniformity can be obtained.

### <Cellulose ether species (Component (B-1))>

Herein, the "cellulose ether species" means one or more cellulose ether species selected from the group consisting of methylcellulose, ethylcellulose, hydroxypropylcellulose, hypromellose, carmellose, carmellose potassium, carmellose calcium, carmellose sodium and croscarmellose sodium, which are compounds in which all or some of hydroxy groups of cellulose form ether bonds; and a salt thereof. The cellulose ether species may be cellulose to which in addition to etherification, further modification such as esterification or crosslink formation as necessary has been applied. The average degree of polymerization, the form (crystal form) and the like of the cellulose ether species are not particularly limited, and the average degree of polymerization is preferably 10 to 10,000.

Specific examples of the cellulose ether species may be used singly, or in combinations of two or more thereof. The alkyl group in the cellulose ether species is not particularly limited, and is preferably a linear or branched C1-C6 alkyl group. The degree of substitution with hydroxyalkoxy groups in the hydroxyalkylcellulose is not particularly limited, and for example, hydroxypropylcellulose includes both non-low substituted hydroxypropylcellulose and low substituted hydroxypropylcellulose. Here, the low substituted hydroxypropylcellulose refers to hydroxypropylcellulose in which the hydroxypropoxy group content determined in a dried state is 5.0 to 16.0% as described in The Japanese Pharmacopoeia, 17th Edition.

From the viewpoint of improvement of content uniformity, the cellulose ether species is preferably one or more selected from the group consisting of methylcellulose, hydroxypropylcellulose, hypromellose, carmellose, carmellose calcium, carmellose sodium and croscarmellose sodium. The hydroxypropylcellulose is preferably low substituted hydroxypropylcellulose. From the viewpoint of ease of production of a pharmaceutical composition (particularly a solid preparation), the cellulose ether species is preferably solid at normal temperature (any temperature in the range of 15 to 25°C) .

Each of these cellulose ether species is a known component. The cellulose ether species may be produced through a known method, or commercially available products may be used. Examples of the commercially available products include ETHOCEL (Dow Chemical Japan Limited), CMEC (Freund Corporation), NS-300 (San-Ei Gen F.F.I., Inc.), ECG-505 (San-Ei Gen F.F.I., Inc.), CELLOGEN (San-Ei Gen F.F.I., Inc.), Ac-Di-Sol (Asahi Kasei Corporation), HEC (Sumitomo Seika Chemicals Co., Ltd.), Hydroxypropylcellulose (Nippon Soda Co., Ltd.), Shin-Etsu AQOAT (Shin-Etsu Chemical Co., Ltd.), METOLOSE 90SH-SR (Shin-Etsu Chemical Co., Ltd.), HPMCP (Shin-Etsu Chemical Co., Ltd.), METOLOSE SM (Shin-Etsu Chemical Co., Ltd.), TC-5 (San-Ei Gen F.F.I., Inc.) and L-HPC (Shin-Etsu Chemical Co., Ltd.).

The content of the cellulose ether species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the cellulose ether species with respect to the total mass of the pharmaceutical composition is preferably 0.5 to 30 mass%, more preferably 1 to 20 mass%, still more preferably 1.5 to 15 mass%, particularly preferably 2 to 10 mass%.

When alkylcelluloses or salts thereof are used as cellulose ether species, the content of the alkylcelluloses or salts thereof with respect to the total mass of the pharmaceutical composition is preferably 0.6 to 22 mass%, more preferably 1.1 to 19 mass%, particularly preferably 3 to 8 mass%, from the viewpoint of improvement of content uniformity.

When hydroxyalkylcelluloses or salts thereof are used as cellulose ether species, the content of the hydroxyalkylcelluloses or salts thereof with respect to the total mass of the pharmaceutical composition is preferably 0.7 to 24 mass%, more preferably 1.2 to 18 mass%, particularly preferably 3 to 8 mass%, from the viewpoint of improvement of content uniformity.

When alkyl(hydroxyalkyl)celluloses, derivatives thereof or salts thereof are used as cellulose ether species, the content of the alkyl(hydroxyalkyl)celluloses, derivatives thereof or salts thereof with respect to the total mass of the pharmaceutical composition is preferably 0.8 to 26 mass%, more preferably 1.3 to 17 mass%, particularly preferably 4 to 9 mass%, from the viewpoint of improvement of content uniformity.

When carboxyalkylcelluloses, derivatives thereof or salts thereof are used as cellulose ether species, the content of the carboxyalkylcelluloses, derivatives thereof or salts thereof with respect to the total mass of the pharmaceutical composition is preferably 0.9 to 28 mass%, more preferably 1.4 to 16 mass%, particularly preferably 1.6 to 9 mass%, from the viewpoint of improvement of content uniformity.

The mass ratio between the content of pemafibrate, a salt thereof or a solvate thereof and the content of the cellulose ether species in the pharmaceutical composition is from the viewpoint of improvement of content uniformity so that the total content of the cellulose ether species with respect to 1 part by mass of a free form of pemafibrate is 3 to 200 parts by mass, preferably 5 to 150 parts by mass, more preferably 10 to 100 parts by mass.

When alkylcelluloses or salts thereof are used as cellulose ether species, the mass ratio between the content of pemafibrate, a salt thereof or a solvate thereof and the content of the alkylcelluloses or salts thereof in the pharmaceutical composition is not particularly limited, and from the viewpoint of improvement of content uniformity, the total content of the alkylcelluloses or salts thereof with respect to 1 part by mass of a free form of pemafibrate is preferably 4 to 160 parts by mass, more preferably 6 to 110 parts by mass, particularly preferably 20 to 60 parts by mass.

When hydroxyalkylcelluloses or salts thereof are used as cellulose ether species, the mass ratio between the content of pemafibrate, a salt thereof or a solvate thereof and the content of the hydroxyalkylcelluloses or salts thereof in the pharmaceutical composition is not particularly limited, and from the viewpoint of improvement of content uniformity, the total content of the hydroxyalkylcelluloses or salts thereof with respect to 1 part by mass of a free form of pemafibrate is preferably 4 to 170 parts by mass, more preferably 7 to 120 parts by mass, still more preferably 20 to 100 parts by mass, particularly preferably 30 to 70 parts by mass.

When alkyl(hydroxyalkyl)celluloses, derivatives thereof or salts thereof are used as cellulose ether species, the mass ratio between the content of pemafibrate, a salt thereof or a solvate thereof and the content of the alkyl(hydroxyalkyl)celluloses, derivatives thereof or salts thereof in the pharmaceutical composition is not particularly limited, and from the viewpoint of improvement of content uniformity, the total content of the alkyl(hydroxyalkyl)celluloses, derivatives thereof or salts thereof with respect to 1 part by mass of a free form of pemafibrate is preferably 4 to 180 parts by mass, more preferably 8 to 130 parts by mass, still more preferably 20 to 100 parts by mass, particularly preferably 40 to 80 parts by mass.

When carboxyalkylcelluloses, derivatives thereof or salts thereof are used as cellulose ether species, the mass ratio between the content of pemafibrate, a salt thereof or a solvate thereof and the content of the carboxyalkylcelluloses, derivatives thereof or salts thereof in the pharmaceutical composition is not particularly limited, and from the viewpoint of improvement of content uniformity, the total content of the carboxyalkylcelluloses, derivatives thereof or salts thereof with respect to 1 part by mass of a free form of pemafibrate is preferably 4 to 190 parts by mass, more preferably 9 to 140 parts by mass, still more preferably 14 to 100 parts by mass, particularly preferably 19 to 90 parts by mass.

### <Starch species (Component (B-2))>

Herein, the "starch species" means one or more selected from the group consisting of starch, hydroxypropyl starch, carboxymethyl starch and a salt thereof. The starch species include those subjected to treatment such as gelatinization or aging. Here, the salt is not particularly limited, and specific examples thereof include alkali metal salts such as sodium salts and potassium salts; and salts with metals of Group 2 elements, such as calcium salts and magnesium salts.

Specific examples of the starch species include starches or salts thereof such as pregelatinized starch, wheat starch, rice starch, corn starch, potato starch, partially pregelatinized starch, wheat flour, rice flour and semi-digested starch; hydroxyalkyl ethers of starch or salts, namelyhydroxypropyl starch; and carboxymethyl ethers of starch or salts thereof such as carboxymethyl starch sodium, and these starches may be used singly, or in combinations of two or more thereof. The alkyl group in the starch species is a specific example of a linear or branched C1-C6 alkyl group.

From the viewpoint of improvement of content uniformity, the starch species is preferably one or more selected from the group consisting of starch and carboxymethyl starch sodium. From the viewpoint of ease of production of a pharmaceutical composition (particularly a solid preparation), the starch species is preferably solid at normal temperature (any temperature in the range of 15 to 25°C).

Each of these starch species is a known component. The starch species may be produced through a known method, or commercially available products may be used. Examples of the commercially available products include LYCATAB PGS (Roquette Japan K.K.), GLYCOLYS (Roquette Japan K.K.), Starch (soluble) (Kishida Chemical Co., Ltd.), Corn Starch (San-Ei Gen F.F.I., Inc.), Potato Starch (JUNSEI CHEMICAL CO., LTD.), HPS-101 (Freund Corporation) and LYCATABC (Roquette Japan K.K.).

The content of the starch species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the starch species with respect to the total mass of the pharmaceutical composition is preferably 0.5 to 50 mass%, more preferably 1 to 40 mass%, still more preferably 1.5 to 30 mass%, particularly preferably 2 to 20 mass%.

When starch is used as starch species, the content of the starch with respect to the total mass of the pharmaceutical composition is preferably 0.6 to 47 mass%, more preferably 1.1 to 38 mass%, particularly preferably 1.6 to 28 mass%, from the viewpoint of improvement of content uniformity.

When carboxyalkyl ethers of starch or salts thereof are used as starch species, the content of the carboxyalkyl ethers of starch or salts thereof with respect to the total mass of the pharmaceutical composition is preferably 0.8 to 45 mass%, more preferably 1.3 to 36 mass%, particularly preferably 1.7 to 26 mass%, from the viewpoint of improvement of content uniformity.

The mass ratio between the content of pemafibrate, a salt thereof or a solvate thereof and the content of the starch species in the pharmaceutical composition is from the viewpoint of improvement of content uniformity, such that the total content of the starch species with respect to 1 part by mass of a free form of pemafibrate is preferably 5 to 400 parts by mass, more preferably 15 to 300 parts by mass, particularly preferably 20 to 200 parts by mass.

When starch is used as starch species, the mass ratio between the content of pemafibrate, a salt thereof or a solvate thereof and the content of the starch in the pharmaceutical composition is not particularly limited, and from the viewpoint of improvement of content uniformity, the total content of the starch with respect to 1 part by mass of a free form of pemafibrate is preferably 7 to 380 parts by mass, more preferably 16 to 280 parts by mass, particularly preferably 30 to 190 parts by mass.

When carboxyalkyl ethers of starch or salts thereof are used as starch species, the mass ratio between the content of pemafibrate, a salt thereof or a solvate thereof and the content of the carboxyalkyl ethers of starch or salts thereof in the pharmaceutical composition is not particularly limited, and from the viewpoint of improvement of content uniformity, the total content of the carboxyalkyl ethers of starch or salts thereof with respect to 1 part by mass of a free form of pemafibrate is preferably 9 to 370 parts by mass, more preferably 17 to 270 parts by mass, particularly preferably 40 to 180 parts by mass.

### <Povidone species (Component (B-3))>

Herein, the "povidone species" means povidone and crospovidone which are polymers of 1-vinyl-2-pyrrolidone. The polymer may be either a non-cross-linked polymer or a cross-linked polymer.

The K value of a linear-chain polymer of 1-vinyl-2-pyrrolidone (povidone) is not particularly limited; the indicated K value is preferably 12 to 90, particularly preferably 25 to 90.

Specific examples of the povidone species include linear-chain polymers of 1-vinyl-2-pyrrolidone, such as povidone (the K value of the povidone is not particularly limited, and the indicated K value is, for example, 12, 17, 25, 30 or 90); and cross-linked polymers of 1-vinyl-2-pyrrolidone, namely crospovidone. These povidones may be used singly, or in combinations of two or more thereof.

From the viewpoint of improvement of content uniformity, the povidone species is one or more selected from the group consisting of povidone and crospovidone, more preferably crospovidone. From the viewpoint of ease of production of a pharmaceutical composition (particularly a solid preparation), the povidone species is preferably solid at normal temperature (any temperature in the range of 15 to 25°C).

Each of these povidone species is a known component. The povidone species may be produced through a known method, or commercially available products may be used. Examples of the commercially available products include Kollidon CL, Kollidon VA64 and Kollidon (each from BASF Japan Ltd.).

The content of the povidone species in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the povidone species with respect to the total mass of the pharmaceutical composition is preferably 0.1 to 20 mass%, more preferably 0.5 to 15 mass%, particularly preferably 1 to 10 mass%.

When linear-chain polymers of 1-vinyl-2-pyrrolidone are used as povidone species, the content of the linear-chain polymers of 1-vinyl-2-pyrrolidone with respect to the total mass of the pharmaceutical composition is preferably 0.2 to 16 mass%, more preferably 0.6 to 14 mass%, particularly preferably 3 to 9 mass%, from the viewpoint of improvement of content uniformity.

When cross-linked polymers of 1-vinyl-2-pyrrolidone are used as povidone species, the content of the cross-linked polymers of 1-vinyl-2-pyrrolidone with respect to the total mass of the pharmaceutical composition is preferably 0.3 to 17 mass%, more preferably 0.7 to 13 mass%, particularly preferably 2 to 8 mass%, from the viewpoint of improvement of content uniformity.

The mass ratio between the content of pemafibrate, a salt thereof or a solvate thereof and the content of the povidone species in the pharmaceutical composition is from the viewpoint of improvement of content uniformity such that the total content of the povidone species with respect to 1 part by mass of a free form of pemafibrate is 1 to 200 parts by mass, more preferably 3 to 150 parts by mass, particularly preferably 5 to 100 parts by mass.

When linear-chain polymers of 1-vinyl-2-pyrrolidone are used as povidone species, the mass ratio between the content of pemafibrate, a salt thereof or a solvate thereof and the content of the linear-chain polymers of 1-vinyl-2-pyrrolidone is not particularly limited, and from the viewpoint of improvement of content uniformity, the total content of the linear-chain polymers of 1-vinyl-2-pyrrolidone with respect to 1 part by mass of a free form of pemafibrate is preferably 1.5 to 190 parts by mass, more preferably 3.5 to 140 parts by mass, particularly preferably 6 to 90 parts by mass.

When cross-linked polymers of 1-vinyl-2-pyrrolidone are used as povidone species, the mass ratio between the content of pemafibrate, a salt thereof or a solvate thereof and the content of the cross-linked polymers of 1-vinyl-2-pyrrolidone is not particularly limited, and from the viewpoint of improvement of content uniformity, the total content of the cross-linked polymers of 1-vinyl-2-pyrrolidone with respect to 1 part by mass of a free form of pemafibrate is preferably 2 to 180 parts by mass, more preferably 4 to 130 parts by mass, particularly preferably 7 to 80 parts by mass.

### <Silicic acid compound (Component (B-4))>

Herein, the "silicic acid compound" includes silicic acid compounds themselves, and salts of silicic acid compounds, namely compounds selected from the group consisting of hydrous magnesium silicate, hydrated silicon dioxide and light anhydrous silicic acid.

Among the silicic acid compounds shown as examples, one or more selected from the group consisting of hydrous magnesium silicate and hydrated silicon dioxide are preferable, from the viewpoint of improvement of content uniformity. From the viewpoint of ease of production of a pharmaceutical composition (particularly a solid preparation), the silicic acid compound is preferably solid at normal temperature (any temperature in the range of 15 to 25°C) .

Each of these silicic acid compounds is a known component. The silicic acid compounds may be produced through a known method, or commercially available products may be used. Examples of the commercially available products include Neusilin A (Fuji Chemical Industries Co., Ltd.), FLORITE (Tomita Pharmaceutical Co., Ltd.), Magnesium Silicate (Tomita Pharmaceutical Co., Ltd.), VEEGUMI GRANULE (Sanyo Chemical Industries, Ltd.), VEEGUMI HV GRANULE (Sanyo Chemical Industries, Ltd.), VEEGUMI K GRANULE (Sanyo Chemical Industries, Ltd.), VEEGUMI F (Sanyo Chemical Industries, Ltd.), SYLYSIA 320 (FUJI SILYSIA CHEMICAL LTD.), SYLYSIA 350 (FUJI SILYSIA CHEMICAL LTD.), SYLYSIA 320TP (FUJI SILYSIA CHEMICAL LTD.), SYLYSIA 320FCP (FUJI SILYSIA CHEMICAL LTD.), MICON FR (Tomita Pharmaceutical Co., Ltd.), Silicon Dioxide (NIPPON AEROSIL CO., LTD.), AEROSIL 300 (NIPPON AEROSIL CO., LTD.), Adsolider 101 (Freund Corporation), Adsolider 102 (Freund Corporation), SYLYSIA (FUJI SILYSIA CHEMICAL LTD.), SYLOSPHERE (FUJI SILYSIA CHEMICAL LTD.), Hydrous Amorphous Silicon Oxide (Tosoh Silica Corporation), Neusilin (Fuji Chemical Industries Co., Ltd.), Diatomaceous Earth (Showa Kako Corporation) and Talc (San-Ei Gen F.F.I., Inc.).

The content of the silicic acid compounds in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the silicic acid compounds with respect to the total mass of the pharmaceutical composition is preferably 0.1 to 20 mass%, more preferably 0.5 to 15 mass%, particularly preferably 1 to 10 mass%.

When one or more selected from the group consisting of a hydrous silicic acid compound and a salt thereof are used as silicic acid compounds, the content of one or more selected from the group consisting of a hydrous silicic acid compound and a salt thereof with respect to the total mass of the pharmaceutical composition is preferably 0.2 to 19 mass%, more preferably 0.6 to 14 mass%, particularly preferably 2 to 6 mass%, from the viewpoint of improvement of content uniformity.

When one or more selected from the group consisting of an anhydrous silicic acid compound and a salt thereof are used as silicic acid compounds, the content of one or more selected from the group consisting of am anhydrous silicic acid compound and a salt thereof with respect to the total mass of the pharmaceutical composition is preferably 0.4 to 17 mass%, more preferably 0.8 to 12 mass%, particularly preferably 4 to 8 mass%, from the viewpoint of improvement of content uniformity.

The mass ratio between the content of pemafibrate, a salt thereof or a solvate thereof and the content of the silicic acid compounds in the pharmaceutical composition is from the viewpoint of improvement of content uniformity such that the total content of the silicic acid compounds with respect to 1 part by mass of a free form of pemafibrate is 1 to 200 parts by mass, more preferably 3 to 150 parts by mass, particularly preferably 5 to 100 parts by mass.

When one or more selected from the group consisting of a hydrous silicic acid compound and a salt thereof, namely hydrous magnesium silicate, are used as silicic acid compounds, the mass ratio between the content of pemafibrate, a salt thereof or a solvate thereof and the content of one or more selected from the group consisting of a hydrous silicic acid compound and a salt thereof is not particularly limited, and from the viewpoint of improvement of content uniformity, the total content of one or more selected from the group consisting of a hydrous silicic acid compound and a salt thereof with respect to 1 part by mass of a free form of pemafibrate is preferably 2 to 160 parts by mass, more preferably 4 to 140 parts by mass, particularly preferably 10 to 90 parts by mass.

When one or more selected from the group consisting of an anhydrous silicic acid compound and a salt thereof, namely light anhydrous silicic acid, are used as silicic acid compounds, the mass ratio between the content of pemafibrate, a salt thereof or a solvate thereof and the content of one or more selected from the group consisting of an anhydrous silicic acid compound and a salt thereof is not particularly limited, and from the viewpoint of improvement of content uniformity, the total content of one or more selected from the group consisting of an anhydrous silicic acid compound and a salt thereof with respect to 1 part by mass of a free form of pemafibrate is preferably 2 to 180 parts by mass, more preferably 4 to 120 parts by mass, particularly preferably 10 to 80 parts by mass.

### <Polyhydric alcohol (Component (B-5))>

Herein, the "polyhydric alcohol" means compounds selected from the group consisting of macrogol, erythritol, xylitol, mannitol, sorbitol, maltitol and lactitol, which are compounds not having two or more cyclic ether structures (for example tetrahydropyran rings) in the molecule while having two or more alcoholic hydroxyl groups, and it may be either a non-polymer or a polymer.

From the viewpoint of ease of production of a pharmaceutical composition (particularly a solid preparation), the polyhydric alcohol is preferably solid at normal temperature (any temperature in the range of 15 to 25°C) .

Specific examples of the sugar alcohol may be used singly, or in combinations of two or more thereof. For these sugar alcohols, various stereoisomers may be present. The steric configuration of the "sugar alcohol" is not particularly limited, and the "sugar alcohol" may be present as a single stereoisomer, or as a mixture of various stereoisomers at any ratio.

From the viewpoint of improvement of content uniformity, the sugar alcohol is one or more selected from the group consisting of erythritol, xylitol, mannitol, sorbitol, maltitol and lactitol, more preferably one or more selected from the group consisting of mannitol, sorbitol and maltitol, particularly preferably mannitol.

Each of these sugar alcohols is a known component. The sugar alcohols may be produced through a known method, or commercially available products may be used. Examples of the commercially available products include Erythritol (San-Ei Gen F.F.I., Inc.), Xylit (Towa Chemical Industry Co., Ltd.), NEOSORB P (Roquette Japan K.K.), Lesys (Towa Chemical Industry Co., Ltd.), Mannit P (Towa Chemical Industry Co., Ltd.), Glycerin (NOF CORPORATION), MALTISORB (Roquette Japan K.K.) and Amalty Syrup (Towa Chemical Industry Co., Ltd.)

The non-sugar alcohol is macrogol (for example macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000) , and these non-sugar alcohols may be used singly, or in combinations of two or more thereof.

From the viewpoint of improvement of content uniformity, the non-sugar alcohol is preferably selected from the group consisting of macrogol 100, macrogol 200, macrogol 300, macrogol 400, macrogol 600, macrogol 1000, macrogol 1500, macrogol 1540, macrogol 4000, macrogol 6000, polyethylene glycol 8000, macrogol 20000 and macrogol 35000, yet more preferably macrogol having an average molecular weight of 100 to 10,000, yet more preferably macrogol having an average molecular weight of 200 to 8,000, particularly preferably macrogol 6000. The average molecular weight of macrogol can be measured in accordance with "Average molecular mass" described in The Japanese Pharmacopoeia, 17th Edition, Pharmaceutical Preparations, Macrogol 400.

Each of these non-sugar alcohols is a known component. The non-sugar alcohols may be produced through a known method, or commercially available products may be used. Examples of the commercially available products include Kollisolv PG (BASF Japan Ltd.), Diethylene Glycol (Nippon Shokubai Co., Ltd.), UNISAFE DPG-R (NOF CORPORATION), Macrogol 200 (Sanyo Chemical Industries, Ltd.), Kollisolv PEG300 (BASF Japan Ltd.), SUPER REFINED PEG 400 (Croda Japan K.K.), CARBOWAX Sentry PEG 600 (Dow Chemical Japan Limited), Macrogol 1000 (NOF CORPORATION), Macrogol 1500 (Sanyo Chemical Industries, Ltd.), CARBOWAX Sentry PEG 1540 (Dow Chemical Japan Limited), Macrogol 4000 (Sanyo Chemical Industries, Ltd.), Macrogol 6000 (Sanyo Chemical Industries, Ltd.), Macrogol 20000 (Sanyo Chemical Industries, Ltd.), NEWPOL PP-2000 (Sanyo Chemical Industries, Ltd.), PRONON 101P (NOF CORPORATION), Kollisolv P124 (BASF Japan Ltd.), PRONON 403P (NOF CORPORATION), NEWDET PE-85 (Sanyo Chemical Industries, Ltd.), PEP-101 (Freund Corporation), Kolliphor P188 (BASF Japan Ltd.), Kolliphor P407 Micro (BASF Japan Ltd.) and UNILUBE DP-950B (NOF CORPORATION).

The content of the polyhydric alcohols in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the polyhydric alcohols with respect to the total mass of the pharmaceutical composition is preferably 0.1 to 99 mass%, more preferably 0.5 to 95 mass%, still more preferably 1 to 90 mass%, particularly preferably 1.5 to 50 mass%.

When sugar alcohols are used as polyhydric alcohols, the content of the sugar alcohols with respect to the total mass of the pharmaceutical composition is preferably 0.2 to 98 mass%, more preferably 0.6 to 94 mass%, particularly preferably 1.1 to 85 mass%, from the viewpoint of improvement of content uniformity.

When non-sugar alcohols are used as polyhydric alcohols, the content of the non-sugar alcohols with respect to the total mass of the pharmaceutical composition is preferably 0.3 to 97 mass%, more preferably 0.7 to 93 mass%, particularly preferably 1.2 to 80 mass%, from the viewpoint of improvement of content uniformity.

When polyalkylene glycols are used as polyhydric alcohols, the content of the polyalkylene glycols with respect to the total mass of the pharmaceutical composition is preferably 0.4 to 96 mass%, more preferably 0.8 to 92 mass%, particularly preferably 1.3 to 75 mass%, from the viewpoint of improvement of content uniformity.

The mass ratio between the content of pemafibrate, a salt thereof or a solvate thereof and the content of the polyhydric alcohols in the pharmaceutical composition is from the viewpoint of improvement of content uniformity such that the total content of the polyhydric alcohols with respect to 1 part by mass of a free form of pemafibrate is 1 to 2,000 parts by mass, more preferably 5 to 1,500 parts by mass, still more preferably 10 to 1,000 parts by mass, particularly preferably 15 to 500 parts by mass.

When sugar alcohols are used as polyhydric alcohols, the mass ratio between the content of pemafibrate, a salt thereof or a solvate thereof and the content of the sugar alcohols in the pharmaceutical composition is not particularly limited, and from the viewpoint of improvement of content uniformity, the total content of the sugar alcohols with respect to 1 part by mass of a free form of pemafibrate is preferably 2 to 1,900 parts by mass, more preferably 6 to 1,450 parts by mass, particularly preferably 12 to 950 parts by mass.

When non-sugar alcohols are used as polyhydric alcohols, the mass ratio between the content of pemafibrate, a salt thereof or a solvate thereof and the content of the non-sugar alcohols in the pharmaceutical composition is not particularly limited, and from the viewpoint of improvement of content uniformity, the total content of the non-sugar alcohols with respect to 1 part by mass of a free form of pemafibrate is preferably 3 to 1,850 parts by mass, more preferably 7 to 1,400 parts by mass, particularly preferably 13 to 900 parts by mass.

When polyalkylene glycols are used as polyhydric alcohols, the mass ratio between the content of pemafibrate, a salt thereof or a solvate thereof and the content of the polyalkylene glycols in the pharmaceutical composition is not particularly limited, and from the viewpoint of improvement of content uniformity, the total content of the polyalkylene glycols with respect to 1 part by mass of a free form of pemafibrate is preferably 4 to 1, 800 parts by mass, more preferably 8 to 1,350 parts by mass, particularly preferably 14 to 850 parts by mass.

### <Alkyl sulfate ester (Component (B-6))>

Herein, the "alkyl sulfate ester" means a lauryl sulfate ester salt, tetradecyl sulfate ester salts, hexadecyl sulfate ester salts and octadecyl sulfate ester salts which is an alkyl sulfate ester salt represented by the following formula:

R-O-SO₃M ... (1)

[wherein R represents a linear or branched saturated or unsaturated C8-C22 hydrocarbon group, and M is an alkali metal such as sodium or potassium; a metal of a Group 2 element such as magnesium or calcium; an ammonium ion; or a C2 or C3 hydroxyalkyl-substituted ammonium such as triethanolammonium] .

These sulfate ester salts may be used singly, or in combinations of two or more thereof.

From the viewpoint of improvement of content uniformity, the alkyl sulfate ester is preferably one or more selected from the group consisting of a lauryl sulfate ester salt, a tetradecyl sulfate ester salt, a hexadecyl sulfate ester salt and an octadecyl sulfate ester salt, more preferably a lauryl sulfate ester salt, particularly preferably sodium lauryl sulfate. From the viewpoint of ease of production of a pharmaceutical composition (particularly a solid preparation), the alkyl sulfate ester is preferably solid at normal temperature (any temperature in the range of 15 to 25°C) .

Each of these alkyl sulfate esters is a known component. The alkyl sulfate esters may be produced through a known method, or commercially available products may be used. Examples of the commercially available products include Kolliphor SLS (BASF Japan Ltd.) .

The content of the alkyl sulfate esters in the pharmaceutical composition is not particularly limited, and can be determined in appropriate consideration of the type of preparation, the sex, age and symptoms of a patient in need of the composition, and the like, but from the viewpoint of improvement of content uniformity, the total amount of the alkyl esters with respect to the total mass of the pharmaceutical composition is preferably 0.1 to 20 mass%, more preferably 0.5 to 15 mass%, particularly preferably 1 to 10 mass%.

The mass ratio between the content of pemafibrate, a salt thereof or a solvate thereof and the content of the alkyl sulfate esters in the pharmaceutical composition is not particularly limited, and from the viewpoint of improvement of content uniformity, the total content of the alkyl sulfate esters with respect to 1 part by mass of a free form of pemafibrate is 1 to 200 parts by mass, more preferably 3 to 150 parts by mass, still more preferably 5 to 100 parts by mass, particularly preferably 5 to 50 parts by mass.

Herein, the dosage form of the "pharmaceutical composition" is a solid preparation, and can be selected according to the use purpose of the pharmaceutical composition. Examples of the dosage form of the pharmaceutical composition include dosage forms described in The Japanese Pharmacopoeia, 17th Edition, General Rules for Preparations. Specific examples of the solid peroral dosage form include preparations such as tablets (e.g. normal tablets, orally disintegrating tablets, chewable tablets, effervescent tablets, dispersion tablets and soluble tablets), capsules, granules (e.g. effervescent granules), powders and pills.

The pharmaceutical composition is preferably a solid preparation from the viewpoint of ease of administration and ease of production. In particular, when the pharmaceutical composition is a solid preparation, production is very easy, but since in general, a solid preparation is produced basically with solid components used in a large amount at normal temperature (any temperature in the range of 15 to 25°C), components are apt to be unevenly mixed and dispersed, so that deterioration in content uniformity is apt to be particularly problematic. On the other hand, the present invention exhibits the following excellent advantage: even a solid preparation has good content uniformity.

The solid preparation is preferably a peroral solid preparation, more preferably a tablet, a capsule, a granule, a dispersion or a pill, particularly preferably a tablet. In addition, the solid preparation is preferably a solid preparation containing a mixture comprising component (A) and component (B).

In addition to the above-described components, pharmaceutically acceptable carriers (additives for pharmaceutical preparation) may be added to the pharmaceutical composition of the present invention depending on its dosage form. Examples of the additives for pharmaceutical preparation include, but are not limited to, diluents, disintegrants, binders, lubricants, plasticizers, film formers, poorly water-soluble polymer substances, antioxidants, flavors and sweetening agents. As specific examples of these additives for pharmaceutical preparation, those described in Japanese Pharmaceutical Excipients Directory 2016 (issued by Yakuji Nippo, Limited), Handbook of Pharmaceutical Excipients, Seventh Edition (issued by Pharmaceutical Press), etc. may be used.

Specific examples of the diluents include inorganic diluents such as anhydrous sodium sulfate, anhydrous dibasic calcium phosphate, sodium chloride, calcium sulfate, calcium monohydrogen phosphate, dibasic calcium phosphate, dibasic sodium phosphate, monobasic potassium phosphate, monobasic calcium phosphate and monobasic sodium phosphate; and organic diluents such as fructose, caramel, agar, paraffin, crystalline cellulose, sucrose, maltose, lactose, lactose monohydrate, white soft sugar, glucose, pullulan, polyoxyethylene hydrogenated castor oil, trehalose, reduced palatinose, maltose, aminoalkyl methacrylate copolymer E, polyvinylacetal diethylaminoacetate and calcium citrate. These diluents may be used singly, or in combinations of two or more thereof.

The total content of the diluents is not particularly limited, and preferably 20 to 99 mass%, more preferably 30 to 97 mass%, with respect to the total mass of the pharmaceutical composition.

Specific examples of the disintegrants include gelatin, sodium hydrogen carbonate, dextrin, dehydroacetic acid and salts thereof and polyoxyethylene hydrogenated castor oil 60. These disintegrants may be used singly, or in combinations of two or more thereof.

Specific examples of the binders include dextrin, pullulan, acacia, agar, gelatin, tragacanth, sodium alginate, aminoalkyl methacrylate copolymer E and polyvinylacetal diethylaminoacetate. These binders may be used singly, or in combinations of two or more thereof.

Specific examples of the lubricants include calcium stearate, magnesium stearate and sodium stearyl fumarate. Theses lubricants may be used singly, or in combinations of two or more thereof.

The total content of the lubricants is not particularly limited, and preferably 0.01 to 15 mass%, more preferably 0.1 to 10 mass%, with respect to the total mass of the pharmaceutical composition.

Specific examples of the plasticizers include sesame oil, castor oil and polysorbate 80 (polyoxyethylene (20) sorbitan oleate ester). These plasticizers may be used singly, or in combinations of two or more thereof.

Specific examples of the film formers include alginic acid or salts thereof such as sodium alginate, carrageenan, xanthan gum and pullulan. These film formers may be used singly, or in combinations of two or more thereof.

Specific examples of the poorly water-soluble polymer substances include carboxyvinyl polymers and aminoalkyl methacrylate copolymers. These substances may be used singly, or in combinations of two or more thereof.

Specific examples of the antioxidants include ascorbic acid, sodium hydrogen sulfite, sodium sulfite, sodium edetate, erythorbic acid, tocopherol acetate, dibutylhydroxytoluene, natural vitamin E, tocopherol and butylhydroxyanisole. These antioxidants may be used singly, or in combinations of two or more thereof.

Specific examples of the flavors include terpenes such as limonene, pinene, camphene, cymene, cineole, citronellol, geraniol, nerol, linalool, menthol, terpineol, rhodinol, borneol, isoborneol, menthone, camphor, eugenol and cinnzeylanol; terpene-containing essential oils such as bitter orange oil, orange oil, peppermint oil, camphor white oil, eucalyptus oil, turpentine oil, lemon oil, ginger oil, clove oil, cinnamon oil, lavender oil, fennel oil, chamomile oil, fermented soybean oil and spearmint oil; and acidulants such as ascorbic acid, tartaric acid, citric acid, malic acid and salts thereof. These flavors may be used singly, or in combinations of two or more thereof.

Examples of the sweetening agents include aspartame, stevia, sucralose, glycyrrhizic acid, thaumatin, acesulfame potassium, saccharin and saccharin sodium, and these sweetening agents may be used singly, or in combinations of two or more thereof.

The pharmaceutical composition of the present invention can be produced through a known method depending on its dosage form.

For example, the pharmaceutical composition, when it is a solid preparation, can be produced through appropriate combination of unit operations such as grinding, mixing, granulation, drying, grain size adjustment, classification, filling, pelletizing and coating. However, the method for producing the same preferably involves a step of mixing component (A) and component (A).

More specifically, for example, when the dosage form of the pharmaceutical composition is a granular preparation such as a granule, a powder or a pill, component (A) and component (B) are mixed with additives for pharmaceutical preparation such as diluents, binders, disintegrants and lubricants in accordance with needs, the mixture is then granulated through a known granulation method such as extrusion granulation, tumbling granulation, agitation granulation, fluidized bed granulation, spray granulation, melt granulation or crushing granulation to obtain a granulated product, and the granulated product is subjected to classification, grain size adjustment and the like in accordance with needs, whereby the pharmaceutical composition can be produced. The obtained granulated product can be coated through a known method with a coating agent etc.

When the dosage form of the pharmaceutical composition is a tablet, component (A) and component (B) are mixed with additives for pharmaceutical preparation such as diluents, binders, disintegrants and lubricants in accordance with needs to obtain a mixture, and the mixture is directly compressed (pelletized) (through a direct powder compression method), or compressed (pelletized) (through a semidry grain compression method, dry granule compression method, wet grain compression method or the like) after the above-described granulated product is subjected to classification, grain size adjustment and the like in accordance with needs, whereby the pharmaceutical composition can be produced. The obtained compressed product (tablet) can be coated through a known method with a coating agent etc.

When the dosage form of the pharmaceutical composition is a capsule, the granulated product or compressed product may be capsulated.

The disease to which the pharmaceutical composition of the present invention is applied is not limited, and the pharmaceutical composition can be widely used for prevention or treatment of diseases against which administration of pemafibrate is known or expected to be effective.

For example, pemafibrate, a salt thereof or a solvate thereof has excellent PPAR-α agonist activity, and exhibits plasma triglyceride concentration reducing action, HDL cholesterol increasing action, etc. Therefore, the pharmaceutical composition of the present invention can be used preferably as an agent for prevention and/or treatment of dyslipidemia (hyperlipidemia, more specifically, for example primary hyperlipidemia and secondary hyperlipidemia), further preferably as an agent for prevention and/or treatment of hypertriglyceridemia, etc.

In addition, pemafibrate, a salt thereof or a solvate thereof is useful for prevention or treatment of NAFLD (non-alcoholic fatty liver disease). Therefore, the pharmaceutical composition of the present invention can also be used as an agent for prevention and/or treatment of NAFLD (more preferably NASH (non-alcoholic steatohepatitis)), etc.

Further, pemafibrate, a salt thereof or a solvate thereof may be used as an agent for treatment of primary biliary cirrhosis, etc.

The administration route of the pharmaceutical composition is not particularly limited, and can be determined in appropriate consideration of the target disease, the type of preparation, the sex, age, symptoms of a patient in need of the composition, and the like, but peroral administration is preferable from the viewpoint of ease of administration. The daily dose of the pharmaceutical composition can be taken as a single dose, or can be divided into 2 to 4 daily administrations, and taken before each meal, between meals, after each meal, before bedtime, or the like.

### Examples

The present invention will next be described in detail by way of Examples, which should not be construed as limiting the invention thereto.

In Test Examples below, measurement was performed through HPLC using an ODS column as a column and an ultraviolet spectrophotometer as a detector.

For pemafibrate used in Test Examples below, the average particle diameters of primary particles were measured in accordance with The Japanese Pharmacopoeia, 17th Edition, Laser Diffraction Measurement of Particle Size, and the results showed that the d50 value was 100 µm or less, and the d90 value was 200 µm or less.

### [Test Example 1]

### Content uniformity evaluation test (1)

The following test was conducted for evaluating the uniformity of the content of pemafibrate in a pharmaceutical composition.

Tablets were produced using the components shown in Table 1 in such a manner that the amounts (mg) of the components per tablet were as shown in Table 1. Specific procedures will be described below.

### (Examples 1 to 5)

Pemafibrate and cellulose ether species were mixed for 30 seconds, lactose monohydrate and microcrystalline cellulose were added, the mixture was mixed for 30 seconds, magnesium stearate was added, and the mixture was mixed for 30 seconds. Thereafter, using a tablet press equipped with a punch having a diameter of 7 mm, the resulting mixture was compressed to obtain 1,000 tablets each having a weight of 120 mg.

### (Comparative Example 1)

Pemafibrate, lactose monohydrate and microcrystalline cellulose were mixed for 30 seconds, magnesium stearate was added, and the mixture was mixed for 30 seconds. Thereafter, using a tablet press equipped with a punch having a diameter of 7 mm, the resulting mixture was compressed to obtain 1,000 tablets each having a weight of 117.6 mg.

From the tablets obtained in Examples and Comparative Example, ten tablets were randomly picked up, and the content of pemafibrate in each tablet was measured through the following method.

One tablet was put in water to crush the tablet, and acetonitrile was then added to obtain a sample solution. The obtained sample solution was analyzed with a HPLC apparatus to measure the pemafibrate-derived peak area. By comparing the pemafibrate-derived peak area for the obtained sample solution to the peak area for a standard solution of pemafibrate with a known concentration, the pemafibrate content per tablet was measured.

From the thus-obtained measured value of the pemafibrate content per tablet, a relative standard deviation (RSD) (%) of the pemafibrate content in the tablet was calculated in accordance with The Japanese Pharmacopoeia, 17th Edition, Content Uniformity Test, and used as an index of variation (degree of uniformity) of the pemafibrate content in the tablet.

Table 1 shows the results.

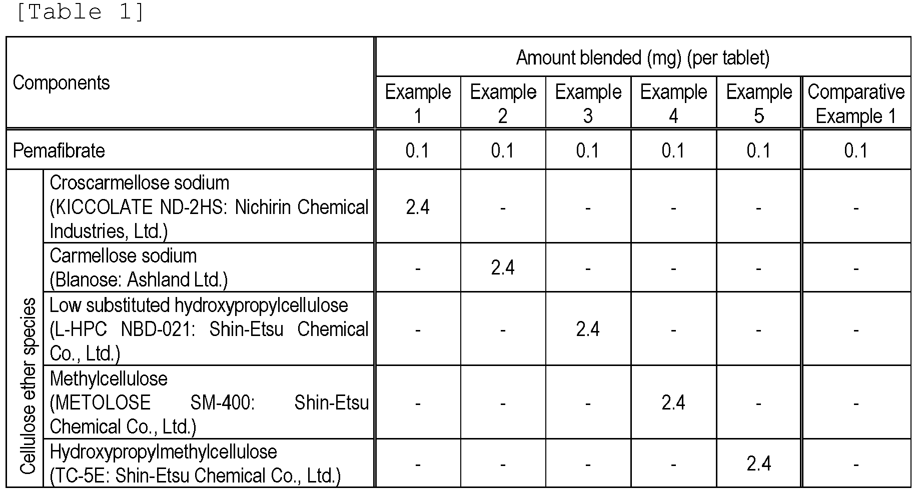

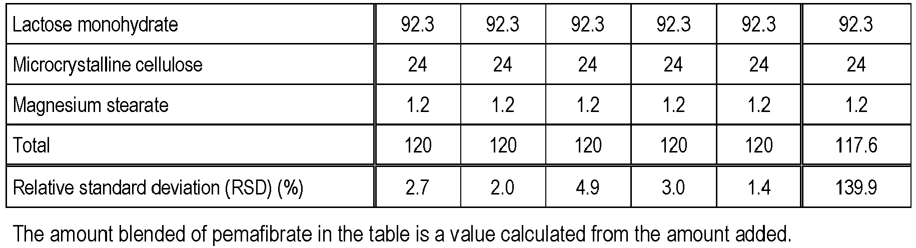

As is apparent from the results shown in Table 1, the tablets of Comparative Example 1 which did not contain cellulose ether species had a relative standard deviation of about 140% and poor uniformity of the content of pemafibrate per tablet.

In contrast, the tablets containing croscarmellose sodium, carmellose sodium, low substituted hydroxypropylcellulose, methylcellulose or hydroxypropylmethylcellulose as cellulose ether species (Examples 1 to 5) all had a small relative standard deviation and good uniformity of the content pemafibrate per tablet. The amount of cellulose ether species added was as small as 2.4 mg (2 mass% with respect to the total mass of the tablet).

The above test results reveal that the content uniformity of pemafibrate in the pharmaceutical composition is improved by incorporating cellulose ether species in a pharmaceutical composition containing pemafibrate, a salt thereof or a solvate thereof.

### [Test Example 2]

### Content uniformity evaluation test (2)

A test was conducted through the same method as in Test Example 1 except that tablets had compositions in which components and the amounts thereof were as shown in Table 2 below.

Table 2 shows the results.

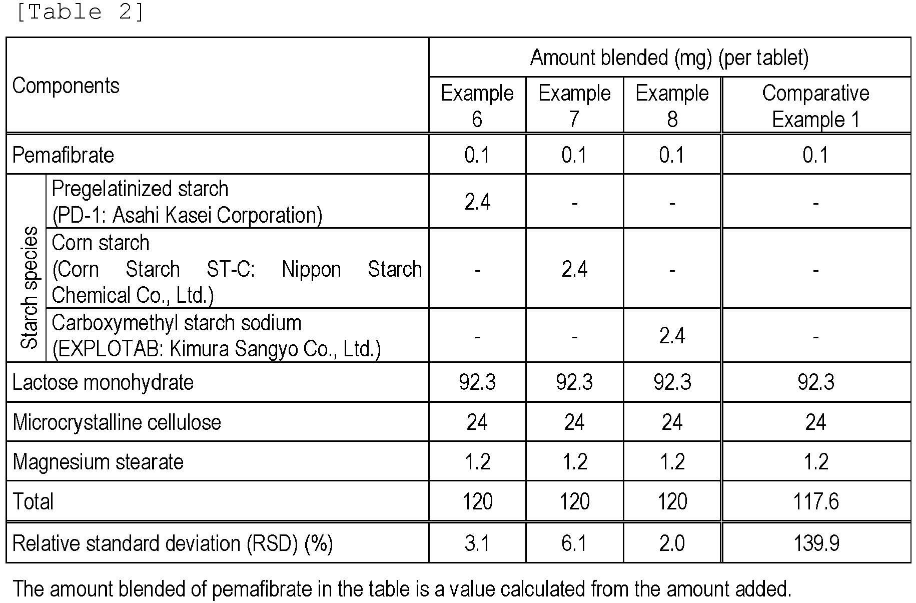

As is apparent from the results shown in Table 2, the tablets containing pregelatinized starch, corn starch or carboxymethyl starch sodium as starch species (Examples 6 to 8) all had a small relative standard deviation and good uniformity of the content pemafibrate per tablet as did the tablets of Examples 1 to 5 containing cellulose ether species in Test Example 1.

The above test results reveal that the content uniformity of pemafibrate in the pharmaceutical composition is improved by incorporating starch species in a pharmaceutical composition containing pemafibrate, a salt thereof or a solvate thereof.

### [Test Example 3]

### Content uniformity evaluation test (3)

A test was conducted through the same method as in Test Example 1 except that tablets had compositions in which components and the amounts thereof were as shown in Table 3 below.

Table 3 shows the results.

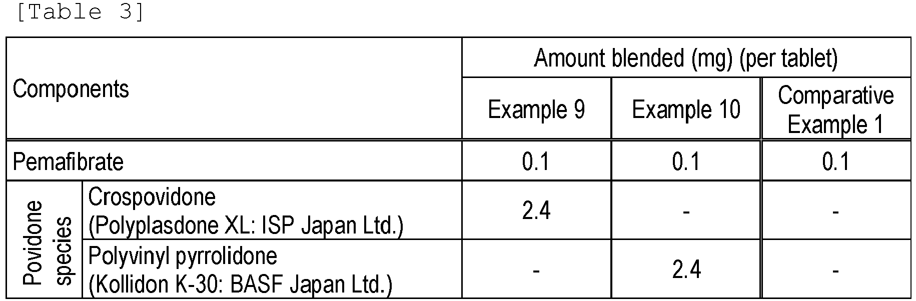

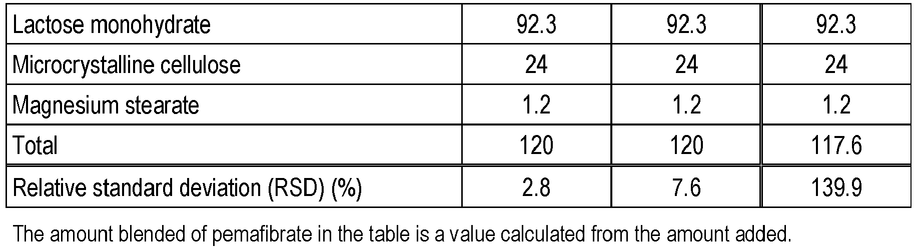

As is apparent from the results shown in Table 3, the tablets containing crospovidone or polyvinyl pyrrolidone as povidone species (Examples 9 and 10) both had a small relative standard deviation and good uniformity of the content pemafibrate per tablet as did the tablets of Examples 1 to 5 containing cellulose ether species in Test Example 1.

The above test results reveal that the content uniformity of pemafibrate in the pharmaceutical composition is improved by incorporating povidone species in a pharmaceutical composition containing pemafibrate, a salt thereof or a solvate thereof.

### [Test Example 4]

### Content uniformity evaluation test (4)

A test was conducted through the same method as in Test Example 1 except that tablets had compositions in which components and the amounts thereof were as shown in Table 4 below.

Table 4 shows the results.

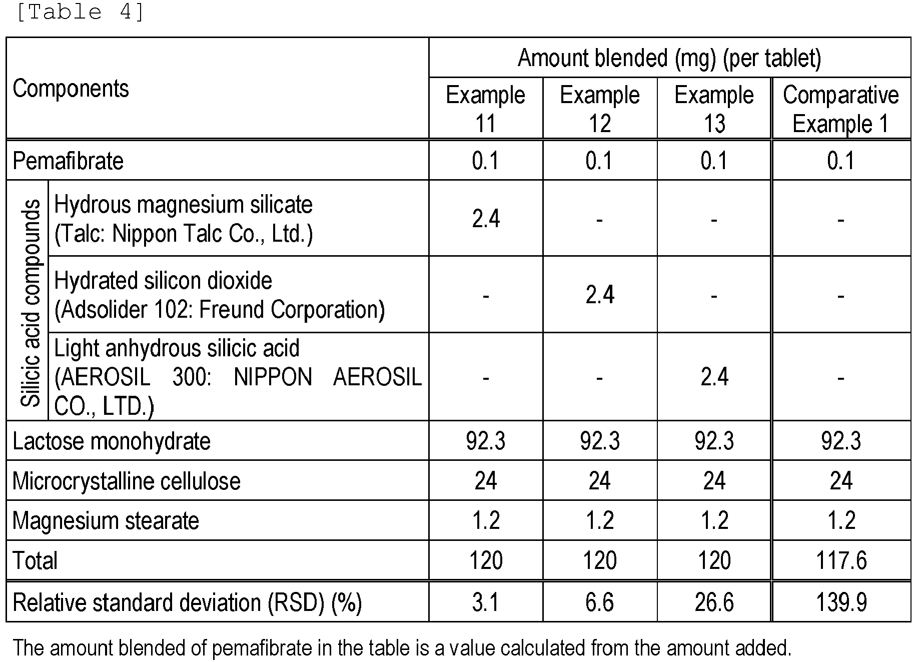

As is apparent from the results shown in Table 4, the tablets containing hydrous magnesium silicate, hydrated silicon dioxide or light anhydrous silicic acid as silicic acid compound (Examples 11 to 13) all had a small relative standard deviation and good uniformity of the content pemafibrate per tablet as did the tablets of Examples 1 to 5 containing cellulose ether species in Test Example 1.

The above test results reveal that the content uniformity of pemafibrate in the pharmaceutical composition is improved by incorporating silicic acid compound in a pharmaceutical composition containing pemafibrate, a salt thereof or a solvate thereof.

### [Test Example 5]

### Content uniformity evaluation test (5)

A test was conducted through the same method as in Test Example 1 except that tablets had compositions in which components and the amounts thereof were as shown in Table 5 below.

Table 5 shows the results.

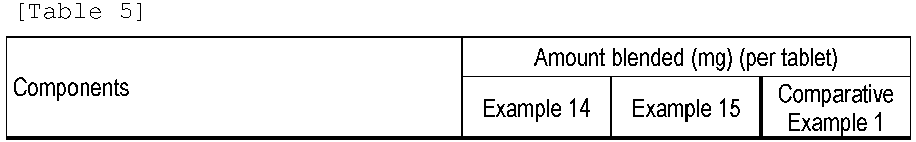

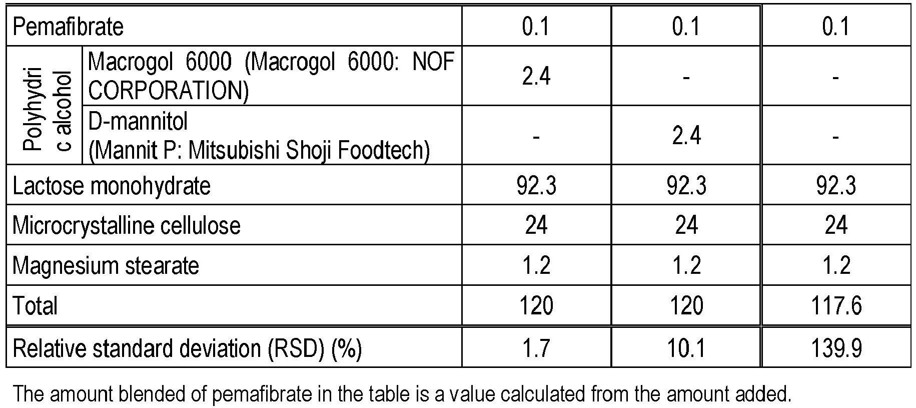

As is apparent from the results shown in Table 5, the tablets containing macrogol or mannitol as polyhydric alcohol (Examples 14 and 15) both had a small relative standard deviation and good uniformity of the content pemafibrate per tablet as did the tablets of Examples 1 to 5 containing cellulose ether species in Test Example 1.

The above test results reveal that the content uniformity of pemafibrate in the pharmaceutical composition is improved by incorporating polyhydric alcohol in a pharmaceutical composition containing pemafibrate, a salt thereof or a solvate thereof.

### [Test Example 6]

### Content uniformity evaluation test (6)

A test was conducted through the same method as in Test Example 1 except that tablets had compositions in which components and the amounts thereof were as shown in Table 6 below.

Table 6 shows the results.

**[Table 6]**

| Components | Amount blended (mg) (per tablet) | |
|---|---|---|
| | Example 21 | Comparative Example 1 |
| Pemafibrate | 0.1 | 0.1 |
| Sodium lauryl sulfate (Sodium Lauryl Sulfate: Wako Pure Chemical Industries, Ltd.) | 2.4 | - |
| Lactose monohydrate | 92.3 | 92.3 |
| Microcrystalline cellulose | 24 | 24 |
| Magnesium stearate | 1.2 | 1.2 |
| Total | 120 | 117.6 |
| Relative standard deviation (RSD) (%) | 1.0 | 139.9 |

| | | |
|---|---|---|
| The amount blended of pemafibrate in the table is a value calculated from the amount added. | | |

As is apparent from the results shown in Table 6, the tablets of Example 16 containing sodium lauryl sulfate as alkyl sulfate ester had a small relative standard deviation and good uniformity of the content pemafibrate per tablet as did the tablets of Examples 1 to 5 containing cellulose ether species in Test Example 1.

The above test results reveal that the content uniformity of pemafibrate in the pharmaceutical composition is improved by incorporating alkyl sulfate ester in a pharmaceutical composition containing pemafibrate, a salt thereof or a solvate thereof.

### [Production Examples 1 to 6]

Tablets containing the components in the amounts (mg) thereof per tablet shown in Tables 7 and 8 are conventionally produced through a wet grain compression method.

**[Table 7]**

| Components | Amount blended (mg) (per tablet) | | |
|---|---|---|---|
| | Production Example 1 | Production Example 2 | Production Example 3 |
| Pemafibrate | 0.1 | 0.4 | 0.1 |
| Lactose monohydrate | q.s. | q.s. | q.s. |
| Magnesium stearate | 1.2 | 1.2 | 1.2 |
| Carmellose sodium | 3 | | 6 |
| Croscarmellose sodium | 1 | | |
| Low substituted hydroxypropylcellulose | | 2 | 4 |
| Crospovidone | | 3 | |
| Carmellose calcium | 2 | | |
| Povidone K25 | | 1 | |
| Ethylcellulose | | 5 | |
| Hydroxyethylmethylcellulose | | 1 | |
| Hypromellose acetate succinate | | | 4 |
| Hypromellose phthalate | | | 6 |
| Hydroxyethylcellulose | 1 | | 2 |
| Hydroxypropylcellulose | 1 | | |
| Hypromellose | | 1 | |
| Methylcellulose | 1 | | |
| Polyvinyl alcohol (partially saponified) | | | 1 |
| Magnesium aluminosilicate | | | 3 |
| Aluminum magnesium silicate | | | 2 |
| Total | 100 mg | 100 mg | 100 mg |

| | | | |
|---|---|---|---|
| The amount blended of pemafibrate in the table is a value calculated from the amount added. | | | |

**[Table 8]**

| Components | Amount blended (mg) (per tablet) | | |
|---|---|---|---|
| | Production Example 4 | Production Example 5 | Production Example 6 |
| Pemafibrate | 0.2 | 0.4 | 0.2 |
| Lactose monohydrate | q.s. | q.s. | q.s. |
| Magnesium stearate | 1.2 | 1.2 | 1.2 |
| Carmellose sodium | | | 1 |
| Croscarmellose sodium | 2 | | 1 |
| Carmellose | | 5 | |
| Carmellose potassium | | | 3 |
| Povidone K90 | 4 | | |
| Copolyvidone | | 3 | |
| Hydroxyethylmethylcellulose | | 9 | |
| Hypromellose acetate succinate | | | 2 |
| Carboxymethylethylcellulose | 5 | | 2 |
| Hydroxypropylcellulose | 1 | | |
| Hypromellose | | 0.5 | |
| Methylcellulose | | | 1 |
| Potato starch | | 15 | |
| Macrogol 400 | | | 2 |
| Macrogol 4000 | | | 2 |
| Macrogol 6000 | | 6 | |
| Polyvinyl alcohol (fully saponified) | 1 | | |
| Calcium silicate | 1 | | |
| Hydrous magnesium silicate | | 5 | |
| Magnesium aluminometasilicate | | 1 | |
| Meglumine | | | 0.5 |
| Bentonite | | | 6 |
| Total | 100 mg | 100 mg | 100 mg |

| | | | |
|---|---|---|---|
| The amount blended of pemafibrate in the table is a value calculated from the amount added. | | | |

### [Production Examples 7 to 12]

Tablets containing the components in the amounts (mg) thereof per tablet shown in Tables 9 and 10 are conventionally produced through a direct powder compression method.

**[Table 9]**

| Components | Amount blended (mg) (per tablet) | | |
|---|---|---|---|
| | Production Example 7 | Production Example 8 | Production Example 9 |
| Pemafibrate | 0.1 | 0.4 | 0.1 |
| Lactose monohydrate | q.s. | q.s. | q.s. |
| Magnesium stearate | 1.2 | 1.2 | 1.2 |
| Pregelatinized starch | 3 | | |
| Wheat starch | 15 | | |
| Rice starch | | 10 | |
| Corn starch | | 20 | |
| Partially pregelatinized starch | | | 3 |
| Wheat flour | | | 10 |
| Hydrated silicon dioxide | | 1 | |
| Hydrous amorphous silicon oxide | | | 0.5 |
| Kaolin | | | 0.4 |
| Talc | 5 | | |
| Sodium lauryl sulfate | 0.3 | | |
| Erythritol | 20 | | |
| Xylitol | | 30 | |
| D-mannitol | | | 40 |
| Propylene glycol | | | 1 |
| Total | 100 mg | 100 mg | 100 mg |

| | | | |
|---|---|---|---|
| The amount blended of pemafibrate in the table is a value calculated from the amount added. | | | |

**[Table 10]**

| Components | Amount blended (mg) (per tablet) | | |
|---|---|---|---|
| | Production Example 10 | Production Example 11 | Production Example 12 |
| Pemafibrate | 0.2 | 0.4 | 0.2 |
| Lactose monohydrate | q.s. | q.s. | q.s. |
| Magnesium stearate | 1.2 | 1.2 | 1.2 |
| Pregelatinized starch | | | 2 |
| Partially pregelatinized starch | | | 6 |
| Rice flour | 10 | | |
| Semi-digested starch | 5 | | |
| Hydroxypropyl starch | | 8 | 1 |
| Carboxymethyl starch sodium (sodium carboxymethyl starch) | | 3 | |
| Light anhydrous silicic acid | 2 | | |
| Heavy anhydrous silicic acid | | 0.3 | |
| Silica | | | 0.1 |
| Natural aluminum silicate | | | 0.8 |
| Synthetic aluminum silicate | | 0.7 | |
| Diatomaceous earth | 0.2 | | |
| D-sorbitol | 50 | | |
| Maltitol | | 60 | |
| Lactitol | | | 70 |
| Polyoxyethylene (105) polyoxypropylene (5) glycol | | 0.5 | |
| Polyoxyethylene (160) polyoxypropylene (30) glycol | | | 2 |
| Total | 100mg | 100 mg | 100 mg |

| | | | |
|---|---|---|---|
| The amount blended of pemafibrate in the table is a value calculated from the amount added. | | | |

### Industrial Applicability

The present invention enables provision of a pharmaceutical composition having excellent homogeneity and containing pemafibrate which exhibits plasma triglyceride concentration reducing action, HDL cholesterol increasing action, etc. The pharmaceutical composition can be used in, for example, pharmaceutical preparation industries.

## Claims

1. A pharmaceutical composition comprising the following components (A) and (B):
(A) pemafibrate, a salt thereof or a solvate thereof; and
(B) one or more selected from the group consisting of the following components (B-1) to (B-6):
(B-1) one or more cellulose ether species selected from the group consisting of methylcellulose, ethylcellulose, hydroxypropylcellulose, hypromellose, carmellose, carmellose potassium, carmellose calcium, carmellose sodium and croscarmellose sodium, and the total content of the cellulose ether species with respect to 1 part by mass of a free form of pemafibrate is 3 to 200 parts by mass;
(B-2) one or more starch species selected from the group consisting of starch, hydroxypropyl starch, carboxymethyl starch and a salt thereof, and the total content of the starch species with respect to 1 part by mass of a free form of pemafibrate is 5 to 400 parts by mass;
(B-3) one or more povidone species selected from the group consisting of povidone and crospovidone, and the total content of the povidone species with respect to 1 part by mass of a free form of pemafibrate is 1 to 200 parts by mass;
(B-4) one or more silicic acid compounds selected from the group consisting of hydrous magnesium silicate, hydrated silicon dioxide and light anhydrous silicic acid, and the total content of the silicic acid compounds with respect to 1 part by mass of a free form of pemafibrate is 1 to 200 parts by mass;
(B-5) one or more polyhydric alcohols selected from the group consisting of macrogol, erythritol, xylitol, mannitol, sorbitol, maltitol and lactitol, and the total content of the polyhydric alcohols with respect to 1 part by mass of a free form of pemafibrate is 1 to 2,000 parts by mass; and
(B-6) one or more alkyl sulfate esters selected from the group consisting of a lauryl sulfate ester salt, tetradecyl sulfate ester salts, hexadecyl sulfate ester salts and octadecyl sulfate ester salts, and the total content of the alkyl sulfate esters with respect to 1 part by mass of a free form of pemafibrate is preferably 1 to 200 parts by mass,
wherein the pharmaceutical composition is a solid preparation.

2. The pharmaceutical composition according to claim 1, wherein the component (B-6) is sodium lauryl sulfate.

3. The pharmaceutical composition according to claim 1 or 2, wherein the dosage form thereof is a tablet, a capsule, a granule, a powder or a pill.

4. A method for improving content uniformity of pemafibrate, a salt thereof or a solvate thereof in a pharmaceutical composition, the method comprising the step of incorporating one or more selected from the group consisting of the following components (B-1) to (B-6):
(B-1) one or more cellulose ether species selected from the group consisting of methylcellulose, ethylcellulose, hydroxypropylcellulose, hypromellose, carmellose, carmellose potassium, carmellose calcium, carmellose sodium and croscarmellose sodium, and the total content of the cellulose ether species with respect to 1 part by mass of a free form of pemafibrate is 3 to 200 parts by mass;
(B-2) one or more starch species selected from the group consisting of starch, hydroxypropyl starch, carboxymethyl starch and a salt thereof, and the total content of the starch species with respect to 1 part by mass of a free form of pemafibrate is 5 to 400 parts by mass;
(B-3) one or more povidone species selected from the group consisting of povidone and crospovidone, and the total content of the povidone species with respect to 1 part by mass of a free form of pemafibrate is 1 to 200 parts by mass;
(B-4) one or more silicic acid compounds selected from the group consisting of hydrous magnesium silicate, hydrated silicon dioxide and light anhydrous silicic acid, and the total content of the silicic acid compounds with respect to 1 part by mass of a free form of pemafibrate is 1 to 200 parts by mass;
(B-5) one or more polyhydric alcohols selected from the group consisting of macrogol, erythritol, xylitol, mannitol, sorbitol, maltitol and lactitol, and the total content of the polyhydric alcohols with respect to 1 part by mass of a free form of pemafibrate is 1 to 2,000 parts by mass; and
(B-6) one or more alkyl sulfate esters selected from the group consisting of a lauryl sulfate ester salt, tetradecyl sulfate ester salts, hexadecyl sulfate ester salts and octadecyl sulfate ester salts, and the total content of the alkyl sulfate esters with respect to 1 part by mass of a free form of pemafibrate is preferably 1 to 200 parts by mass,
wherein the pharmaceutical composition is a solid preparation,
in a pharmaceutical composition comprising pemafibrate, a salt thereof or a solvate thereof.

## Patentansprüche

1. Pharmazeutische Zusammensetzung, umfassend die folgenden Bestandteile (A) und (B):
(A) Pemafibrat, ein Salz desselben oder ein Solvat desselben und
(B) einen oder mehrere Bestandteile, ausgewählt aus der Gruppe, bestehend aus den folgenden Bestandteilen (B-1) bis (B-6):
(B-1) eine oder mehrere Celluloseether-Arten, ausgewählt aus der Gruppe, bestehend aus Methylcellulose, Ethylcellulose, Hydroxypropylcellulose, Hypromellose, Carmellose, Carmellose-Kalium, Carmellose-Calcium, Carmellose-Natrium und Croscarmellose-Natrium, und der Gesamtgehalt der Celluloseether-Arten beträgt 3 bis 200 Massenteile, bezogen auf 1 Massenteil der freien Form von Pemafibrat;
(B-2) eine oder mehrere Stärke-Arten, ausgewählt aus der Gruppe, bestehend aus Stärke, Hydroxypropylstärke, Carboxymethylstärke und einem Salz derselben, und der Gesamtgehalt der Stärke-Arten beträgt 5 bis 400 Massenteile, bezogen auf 1 Massenteil der freien Form von Pemafibrat,
(B-3) eine oder mehrere Povidon-Arten, ausgewählt aus der Gruppe, bestehend aus Povidon und Crospovidon, und der Gesamtgehalt der Povidon-Arten beträgt 1 bis 200 Massenteile, bezogen auf 1 Massenteil der freien Form von Pemafibrat;
(B-4) eine oder mehrere Kieselsäureverbindungen, ausgewählt aus der Gruppe, bestehend aus wasserhaltigem Magnesiumsilikat, hydratisiertem Siliciumdioxid und leichter wasserfreier Kieselsäure, und der Gesamtgehalt der Kieselsäureverbindungen beträgt 1 bis 200 Massenteile, bezogen auf Massenteil der freien Form von Pemafibrat;
(B-5) einen oder mehrere mehrwertige Alkohole, ausgewählt aus der Gruppe, bestehend aus Macrogol, Erythritol, Xylitol, Mannitol, Sorbitol, Maltitol und Lactitol, und der Gesamtgehalt der mehrwertigen Alkohole beträgt 1 bis 2000 Massenteile, bezogen auf 1 Massenteil der freien Form von Pemafibrat; und
(B-6) einen oder mehrere Alkylsulfatester, ausgewählt aus der Gruppe, bestehend aus einem Laurylsulfatestersalz, Tetradecylsulfatestersalzen, Hexadecylsulfatestersalzen und Octadecylsulfatestersalzen, und der Gesamtgehalt der Alkylsulfatester beträgt bevorzugt 1 bis 200 Massenteile bezogen auf 1 Massenteil der freien Form von Pemafibrat, wobei die pharmazeutische Zusammensetzung eine feste Zubereitung ist.

2. Pharmazeutische Zusammensetzung nach Anspruch 1, wobei der Bestandteil (B-6) Natriumlaurylsulfat ist.

3. Pharmazeutische Zusammensetzung nach Anspruch 1 oder 2, wobei die Dosierungsform derselben eine Tablette, eine Kapsel, ein Granulat, ein Pulver oder eine Pille ist.

4. Verfahren zur Verbesserung der Gleichförmigkeit des Gehalts an Pemafibrat, eines Salzes desselben oder eines Solvats desselben in einer pharmazeutischen Zusammensetzung, wobei das Verfahren den Schritt der Aufnahme eines oder mehrerer Bestandteile, ausgewählt aus der Gruppe, bestehend aus den folgenden Bestandteilen (B-1) bis (B-6):
(B-1) eine oder mehrere Celluloseether-Arten, ausgewählt aus der Gruppe, bestehend aus Methylcellulose, Ethylcellulose, Hydroxypropylcellulose, Hypromellose, Carmellose, Carmellose-Kalium, Carmellose-Calcium, Carmellose Natrium und Croscarmellose-Natrium, und der Gesamtgehalt der Celluloseether-Arten beträgt 3 bis 200 Massenteile, bezogen auf 1 Massenteil der freien Form von Pemafibrat;
(B-2) eine oder mehrere Stärke-Arten, ausgewählt aus der Gruppe, bestehend aus Stärke, Hydroxypropylstärke, Carboxymethylstärke und einem Salz derselben, und der Gesamtgehalt der Stärke-Arten beträgt 5 bis 400 Massenteile, bezogen auf 1 Massenteil der freien Form von Pemafibrat,
(B-3) eine oder mehrere Povidon-Arten, ausgewählt aus der Gruppe, bestehend aus Povidon und Crospovidon, und der Gesamtgehalt der Povidon-Arten beträgt 1 bis 200 Massenteile, bezogen auf 1 Massenteil der freien Form von Pemafibrat;
(B-4) eine oder mehrere Kieselsäureverbindungen, ausgewählt aus der Gruppe, bestehend aus wasserhaltigem Magnesiumsilikat, hydratisiertem Siliciumdioxid und leichter wasserfreier Kieselsäure, und der Gesamtgehalt der Kieselsäureverbindungen beträgt 1 bis 200 Massenteile, bezogen auf Massenteil der freien Form von Pemafibrat;
(B-5) einen oder mehrere mehrwertige Alkohole, ausgewählt aus der Gruppe, bestehend aus Macrogol, Erythritol, Xylitol, Mannitol, Sorbitol, Maltitol und Lactitol, und der Gesamtgehalt der mehrwertigen Alkohole beträgt 1 bis 2000 Massenteile, bezogen auf 1 Massenteil der freien Form von Pemafibrat; und
(B-6) einen oder mehrere Alkylsulfatester, ausgewählt aus der Gruppe, bestehend aus einem Laurylsulfatestersalz, Tetradecylsulfatestersalzen, Hexadecylsulfatestersalzen und Octadecylsulfatestersalzen, und der Gesamtgehalt der Alkylsulfatester beträgt bevorzugt 1 bis 200 Massenteile bezogen auf 1 Massenteil der freien Form von Pemafibrat, wobei die pharmazeutische Zusammensetzung eine feste Zubereitung ist, in eine pharmazeutische Zusammensetzung umfasst, die Pemafibrat ein Salz desselben oder ein Solvat desselben umfasst.

## Revendications

1. Composition pharmaceutique comprenant les composants (A) et (B) suivants :
(A) pémafibrate, un sel de celui-ci ou un solvate de celui-ci ; et
(B) un ou plusieurs éléments sélectionnés parmi le groupe constitué des composants suivants (B-1) à (B-6) :
(B-1) une ou plusieurs espèces d'éther de cellulose sélectionnées parmi le groupe constitué de méthylcellulose, éthylcellulose, hydroxypropylcellulose, hypromellose, carmellose, carmellose potassique, carmellose calcique, carmellose sodique et croscarmellose sodique, et la teneur totale des espèces d'éther de cellulose par rapport à 1 partie en masse d'une forme libre de pémafibrate est de 3 à 200 parties en masse ;
(B-2) une ou plusieurs espèces d'amidon sélectionnées parmi le groupe constitué d'amidon, amidon hydroxypropylique, amidon carboxyméthylique et d'un sel de ceux-ci, et la teneur totale des espèces d'amidon par rapport à 1 partie en masse d'une forme libre de pémafibrate est de 5 à 400 parties en masse ;
(B-3) une ou plusieurs espèces de povidone sélectionnées parmi le groupe constitué de povidone et crospovidone, et la teneur totale des espèces de povidone par rapport à 1 partie en masse d'une forme libre de pémafibrate est de 1 à 200 parties en masse ;
(B-4) un ou plusieurs composés d'acide silicique sélectionnés parmi le groupe constitué de silicate de magnésium hydrique, dioxyde de silicium hydraté et acide silicique anhydre léger, et la teneur totale des composés d'acide silicique par rapport à 1 partie en masse d'une forme libre de pémafibrate est de 1 à 200 parties en masse ;
(B-5) un ou plusieurs alcools polyhydriques sélectionnés parmi le groupe constitué de macrogol, érythritol, xylitol, mannitol, sorbitol, maltitol et lactitol, et la teneur totale des alcools polyhydriques par rapport à 1 partie en masse d'une forme libre de pémafibrate est de 1 à 2000 parties en masse ; et
(B-6) un ou plusieurs esters d'alkylsulfate sélectionnés parmi le groupe constitué d'un sel d'ester de laurylsulfate, sels d'ester de tétradécylsulfate, sels d'ester d'hexadecylsulfate et sels d'ester d'octadécylsulfate, et la teneur totale des esters d'alkylsulfate par rapport à 1 partie en masse d'une forme libre de pémafibrate est préférentiellement de 1 à 200 parties en masse,
dans laquelle la composition pharmaceutique est une préparation solide.

2. Composition pharmaceutique selon la revendication 1, dans laquelle le composant (B-6) est du laurylsulfate de sodium.

3. Composition pharmaceutique selon la revendication 1 ou 2, dans laquelle sa forme de dosage est un comprimé, une capsule, un granulé, une poudre ou une pilule.

4. Procédé d'amélioration d'uniformité de teneur de pémafibrate, d'un sel de celui-ci ou d'un solvate de celui-ci dans une composition pharmaceutique, le procédé comprenant l'étape d'incorporation d'un ou plusieurs éléments sélectionnés parmi le groupe constitué des composants suivants (B-1) à (B-6) :
(B-1) une ou plusieurs espèces d'éther de cellulose sélectionnées parmi le groupe constitué de méthylcellulose, éthylcellulose, hydroxypropylcellulose, hypromellose, carmellose, carmellose potassique, carmellose calcique, carmellose sodique et croscarmellose sodique, et la teneur totale des espèces d'éther de cellulose par rapport à 1 partie en masse d'une forme libre de pémafibrate est de 3 à 200 parties en masse ;
(B-2) une ou plusieurs espèces d'amidon sélectionnées parmi le groupe constitué d'amidon, amidon hydroxypropylique, amidon carboxyméthylique et d'un sel de ceux-ci, et la teneur totale des espèces d'amidon par rapport à 1 partie en masse d'une forme libre de pémafibrate est de 5 à 400 parties en masse ;
(B-3) une ou plusieurs espèces de povidone sélectionnées parmi le groupe constitué de povidone et crospovidone, et la teneur totale des espèces de povidone par rapport à 1 partie en masse d'une forme libre de pémafibrate est de 1 à 200 parties en masse ;
(B-4) un ou plusieurs composés d'acide silicique sélectionnés parmi le groupe constitué de silicate de magnésium hydrique, dioxyde de silicium hydraté et acide silicique anhydre léger, et la teneur totale des composés d'acide silicique par rapport à 1 partie en masse d'une forme libre de pémafibrate est de 1 à 200 parties en masse ;
(B-5) un ou plusieurs alcools polyhydriques sélectionnés parmi le groupe constitué de macrogol, érythritol, xylitol, mannitol, sorbitol, maltitol et lactitol, et la teneur totale des alcools polyhydriques par rapport à 1 partie en masse d'une forme libre de pémafibrate est de 1 à 2000 parties en masse ; et
(B-6) un ou plusieurs esters d'alkylsulfate choisis parmi le groupe constitué d'un sel d'ester de laurylsulfate, sels d'ester de tétradécylsulfate, sels d'ester d'hexadecylsulfate et sels d'ester d'octadécylsulfate, et la teneur totale des esters d'alkylsulfate par rapport à 1 partie en masse d'une forme libre de pémafibrate est préférentiellement de 1 à 200 parties en masse,
dans lequel la composition pharmaceutique est une préparation solide,
dans une composition pharmaceutique comprenant du pémafibrate, un sel de celui-ci ou un solvate de celui-ci .
